# EUROPEAN PATENT SPECIFICATION

(11) **EP 3 166 942 B1**
(45) Date of publication and mention of the grant of the patent: **13.09.2017**
(21) Application number: 15738305.0
(22) Date of filing: 10.07.2015
(51) Int. Cl.: C07D 471/04, A61K 31/437, A61P 7/02

(54) **PROCESS FOR PREPARING APIXABAN**
VERFAHREN ZUR HERSTELLUNG VON APIXABAN
PROCÉDÉ DE PRÉPARATION D'APIXABAN

(30) Priority: 11.07.2014 EP 14382272
(43) Date of publication of application: 17.05.2017
(73) Proprietor: Laboratorios Lesvi, S.L., 08970 Sant Joan Despí - Barcelona (ES)
(72) Inventor: DALMASES BARJOAN, Pere, E-08980 Sant Feliu de Llobregat (Barcelona) (ES); HUGUET CLOTET, Juan, E-08970 Sant Joan Despí (Barcelona) (ES); NAVARRO MUÑOZ, Isabel, E-08970 Sant Joan Despí (Barcelona) (ES)
(74) Representative: ABG Patentes, S.L.
(86) International application number: PCT/EP2015/065838
(87) International publication number: WO 2016/005560

(56) References cited:
- WO-A1-2013/119328
- WO-A2-2004/099130
- WO-A2-2007/001385

## Description

### FIELD OF THE INVENTION

The present invention refers to an efficient and environmentally friendly process for the preparation of 1-(4-methoxyphenyl)-7-oxo-6-[4-(2-oxopiperidin-1-yl)phenyl]-4,5,6,7-tetrahydro-1*H*-pyrazolo[3,4-c]pyridine-3-carboxamide and pharmaceutically acceptable salts thereof with high yield and purity suitable for industrial scale applications.

### BACKGROUND OF THE INVENTION

1-(4-methoxyphenyl)-7-oxo-6-[4-(2-oxopiperidin-1-yl)phenyl]-4,5,6,7-tetrahydro-1*H-*pyrazolo [3,4-c]pyridine-3-carboxamide, also known as apixaban (compound I) is an anticoagulant and antithrombotic Factor Xa inhibitor, developed by Bristol-Myers Squibb for the prevention of venous thromboembolism and the prevention of stroke in atrial fibrillation and is depicted below. Apixaban is marketed under the tradename Eliquis®.

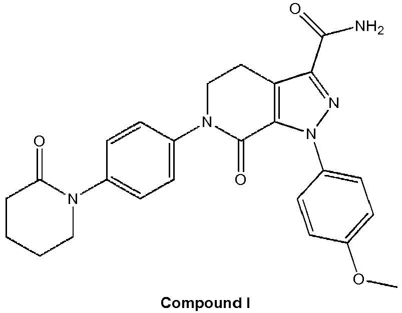

A number of methods of synthesis of apixaban have been reported up to date. Apixaban was first disclosed by the European patent EP1427415 to Bristol-Myers Squibb, which describes a synthetic approach to the preparation of apixaban, similar molecules and also pharmaceutical composition containing them. The process described in the aforementioned patent involved the use of a large number of steps providing apixaban in very low yields, which had to be purified by means of column chromatography, an unsuitable method for industrial scale applications. In addition, this method presents some disadvantages, in particular, long reaction times and the use of Class 1 and Class 2 solvents (solvents that should be avoided and limited in pharmaceutical products) which are toxic and environmentally hazardous.

International application WO03/049681 to Bristol-Myers Squibb Company relates generally to processes for the preparation of 4,5-dihydro-pyrazolo [3,4-c]pyrid-2-ones such pyrazolo-pyridinones being useful as factor Xa inhibitors and intermediates for the synthesis thereof. The conditions for preparing the intermediates for the preparation of apixaban involve the use of expensive and potentially hazardous reagents.

International application WO2007/001385 to Bristol-Myers Squibb Company describes general processes for the preparation of 4,5-dihydro-pyrazolo [3,4-c]pyrid-2-ones and intermediates. Particularly, the application patent describes the preparation of apixaban, as depicted below in the scheme.

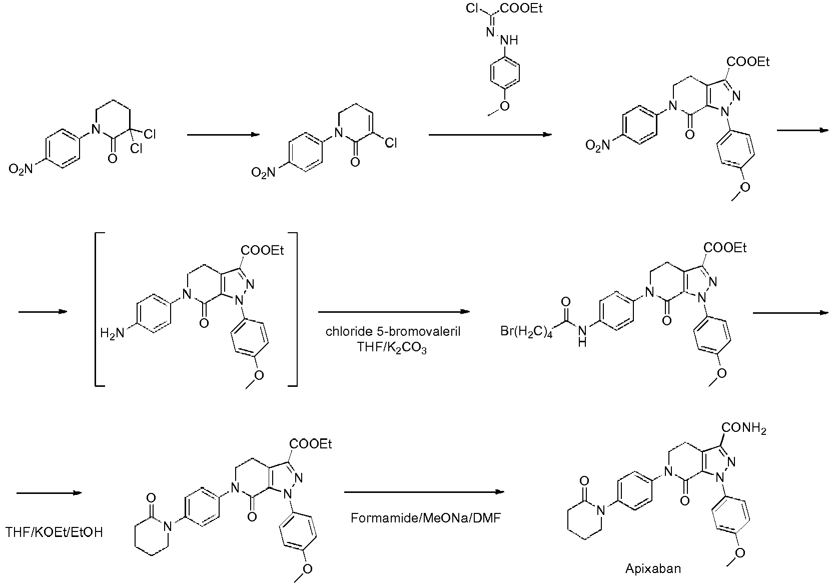

Apixaban is provided in two different polymorphic forms; however no information regarding polymorphic or chemical purity of apixaban is disclosed. To date the prior-art provides multi-step, complex and expensive methods for obtaining apixaban in high yield. These disadvantages also increase the cost of the final apixaban and the pharmaceutical compositions containing it. In view of the pharmaceutical value of this compound, it is important to obtain an efficient and safe process for the preparation of apixaban and salts thereof, which can be applied at an industrial scale with low energy and costs and high yields. Therefore, there is a need to develop an improved industrially feasible and more economical process for the preparation of apixaban, which is more efficient, which can provide apixaban in high purity and yield and which can be easily performed at industrial scale.

### BRIEF DESCRIPTION OF THE INVENTION

The present invention provides an efficient process for manufacturing apixaban in high yields and applicable at industrial scale. This process also allows obtaining apixaban without requiring laborious and unfeasible purification steps and yielding a high purity product which complies with pharmaceutical standards. In addition the process of the present invention is environmentally friendly.

Accordingly, the present invention provides a process for manufacturing apixaban (compound I) or a pharmaceutically acceptable salt thereof, wherein the process comprises at least the following steps:
a) Contacting ethyl 6-(4-(5-bromopentanamido)phenyl)-1-(4-methoxyphenyl)-7-oxo-4,5,6,7-tetrahydro-1*H*-pyrazolo[3,4-*c*]pyridine-3-carboxylate (compound II) with formamide in the presence of a base to yield 1-(4-methoxyphenyl)-7-oxo-6-(4-(2-oxopiperidin-1-yl)phenyl)-4,5,6,7-tetrahydro-1*H*-pyrazolo[3,4-*c*]pyridine-3-carboxamide (compound I); 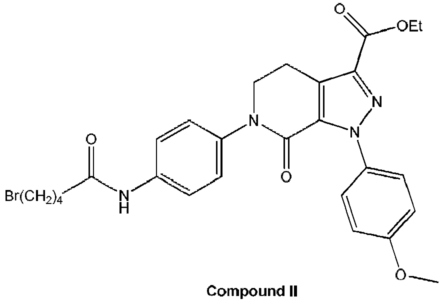 wherein the ratio of the base to the compound II, is from 2:1 (mol/mol) to 10:1 (mol/mol).
b) isolating the apixaban obtained in step a)
c) optionally purifying apixaban of step a) or b) by means of conventional purification techniques.
d) optionally, converting the apixaban obtained in step a) or b) into a pharmaceutically acceptable salt or co-crystal thereof.

A second aspect of the present disclosure relates to a pharmaceutical composition comprising a therapeutically effective amount of apixaban prepared according to the first aspect of the present invention together with appropriate amount of pharmaceutically acceptable excipients or carriers.

A third aspect of the present disclosure relates to the use of the pharmaceutical composition of the second aspect of the present disclosure for use in a medicament.

A forth aspect of the present disclosure relates to the pharmaceutical composition of the second aspect of the present disclosure to prevent venous thromboembolism in adults following a hip or knee replacement operation and/or to prevent stroke and blood clots in other organs in adults with atrial fibrillation

### DEFINITIONS

The term "base" as used herein refers to an alkoxide base. Suitable alkoxide bases include linear or branched alkoxides of metals (metal alkoxides), ammonium (ammonium alkoxides), boron (boron alkoxides) and silicon (silicon alkoxides). Metal alkoxides include, linear or branched alkoxides of metals selected from alkaline metals, alkaline earth metal or a transition metal. Examples of alkoxide bases are sodium methoxide, sodium ethoxide, sodium propoxide, sodium isopropoxide, sodium butoxide, sodium isobutoxide, sodium sec-butoxide, sodium tert-butoxide, lithium methoxide, lithium ethoxide, potassium methoxide, potassium ethoxide, potassium propoxide, potassium isopropoxide, potassium butoxide, potassium isobutoxide, potassium sec-butoxide, and potassium tert-butoxide, calcium methoxide, calcium ethoxide, magnesium methoxide, magnesium ethoxide, barium methoxide, barium ethoxide, aluminum methoxide, aluminum ethoxide, titanium methoxide, titanium ethoxide, zirconium methoxide, zirconium ethoxide, ammonium methoxide, ammonium ethoxide, silicon methoxide, silicon ethoxide, boron methoxide, boron ethoxide.

As used herein the term "organic solvent" refers to an organic molecule capable of at least partially dissolving another substance (i.e., the solute). Organic solvents may be liquids at room temperature. Examples of organic solvents that may be used for the present invention include, but are not limited to: hydrocarbon solvents (e.g., n-pentane, n-hexane, n-heptane, n-octane, paraffin, cyclohexane, methylcyclohexane, decahydronaphthalene, mineral oil, crude oils, etc.) which also includes aromatic hydrocarbon solvents (e.g., benzene, toluene, o-xylene, m-xylene, and p-xylene), halogenated hydrocarbon solvents (e.g., carbon tetrachloride, 1,2-dichloroethane, dichloromethane, chloroform, etc.), ester solvents (e.g., ethyl formate, methyl acetate, ethyl acetate, isopropyl acetate, ethyl malonate, etc.), ketone solvents (e.g., acetone, methyl ethyl ketone, cyclohexanone, cyclopentanone, etc.), ether solvents (e.g., diethyl ether, dipropyl ether, diphenyl ether, tetrahydrofuran, 1,4-dioxane, etc.), amine solvents (e.g., propyl amine, diethylamine, triethylamine, aniline, pyridine), alcohol solvents (e.g., methanol, ethanol, 1-propanol, 1-butanol, 1-octanol, benzyl alcohol, phenol, trifluoroethanol, glycerol, ethylene glycol, propylene glycol, m-cresol, etc.), carbon disulfide, nitrobenzene, N,N-dimethylformamide (DMF), N,N,-dimethylacetamide, dimethyl sulfoxide, N-methyl-2-pyrrolidone, acetonitrile, silicone solvents (e.g., silicone oils, polysiloxanes, cyclosilicones). In some embodiments, the organic solvent may be formed by the combination of two or more organic solvents.

The term "polar solvent" as used herein means a solvent that tends to interact with other compounds or itself through acid-base interactions, hydrogen bonding, dipole-dipole interactions, or by dipole-induced dipole interactions.

The term "non-polar solvent" as used herein means a solvent that is not a polar solvent. Non-polar solvents interact with other compounds or themselves predominantly through dispersion forces. Non-polar solvents interact with polar solvents mainly through dipole-induced dipole interactions or through dispersion forces. Non-limiting examples of these solvents include toluene, xylene, n-heptane, octane, isooctane, cyclohexane, pentane and 1,4-dioxane.

The term "aprotic solvent" as used herein means any molecular solvent which cannot donate H⁺, i.e. a compound not having labile hydrogens. Examples of aprotic solvents that may be used for the present invention include, but are not limited to: Tetrahydrofuran (THF), 2-methyl tetrahydrofuran, *N*,*N*-dimethylformamide, *N*,*N*,-dimethylacetamide, dimethyl sulfoxide, N-methyl-2-pyrrolidone, acetonitrile, acetone, ethyl acetate, isopropyl acetate, toluene, methyl cyclohexane, acetonitrile, methyl ethyl ketone (MEK), and methyl isobutyl ketone (MiBK).

As used herein, the term "antisolvent" refers to a solvent in which the apixaban has a solubility of less than 0.1 g/L. Suitable antisolvents for the purification process of apixaban include alcohols, ketones, ethers, esters, hydrocarbons and any mixtures thereof.

The term "solvate" refers to a molecular complex comprising apixaban or apixaban derivate and a stoichiometric or non-stoichiometric amount of one or more solvent molecules (e.g. acetone).

The term "hydrate" refers to a molecular complex comprising apixaban or apixaban derivate and a stoichiometric or non-stoichiometric amount of water.

As used herein, the term "washing" refers to the process of purifying a solid mass (e.g., crystals) by passing a liquid over and/or through the solid mass, as to remove undesirable soluble matter. The process includes passing a solvent, such as distilled water, over and/or through a precipitate obtained from filtering, decanting, or a combination thereof. For example, in one embodiment of the invention, washing includes contacting solids with solvent or solvent mixture, vigorously stirring (e.g., for two hours), and filtering. The solvent can be water, can be an aqueous solvent system, or can be an organic solvent system. Additionally, the washing can be carried out with the solvent having any suitable temperature. For example, the washing step can be carried out with a solvent having a temperature between about 0 °C and about 100 °C. The term "conventional isolation techniques" as used herein refers to the process wherein an isolated product can be obtained, which can be carried out on an industrial scale such as solvent extraction, filtration, distillation, slurring, washing, phase separation, evaporation, centrifugation or crystallization.

As used herein, the term, "solvent extraction" refers to the process of separating components of a mixture by using a solvent which possesses greater affinity for one component, and may therefore separate said one component from at least a second component which is less miscible than said one component with said solvent.

The term "filtration" refers to the act of removing solid particles greater than a predetermined size from a feed comprising a mixture of solid particles and liquid. The expression "filtrate" refers to the mixture less the solid particles removed by the filtration process. It will be appreciated that this mixture may contain solid particles smaller than the predetermined particle size. The expression "filter cake" refers to residual solid material remaining on a feed side of a filtration element.

The term "evaporation" refers to the change in state of solvent from liquid to gas and removal of that gas from the reactor. Various solvents may be evaporated during the synthetic route disclosed herein. As known to those of skilled in the art, each solvent may have a different evaporation time and/or temperature.

As used herein, the term "slurrying" refers to any process which employs a solvent to wash, suspend or disperse a crude solid product.
The term "phase separation" refers to a solution or mixture having at least two physically distinct regions.

The term "crystallization" refers to any method known to a person skilled in the art wherein products are obtained in crystal form such as crystallization from single solvent or combination of solvents by dissolving the compound optionally at elevated temperature and precipitating the compound by cooling the solution or removing solvent from the solution or both. It further includes methods such as solvent/antisolvent crystallization.

The term "purification" as used herein refers to the process of rendering a drug substance clean of foreign elements whereby a purified drug substance can be obtained. The term "industrial purification" refers to purifications which can be carried out on an industrial scale such as solvent extraction, filtration, slurring, washing, phase separation, evaporation, centrifugation or crystallization.

### BRIEF DESCRIPTION OF THE DRAWINGS

Examples of the present invention are illustrated with the following drawings:

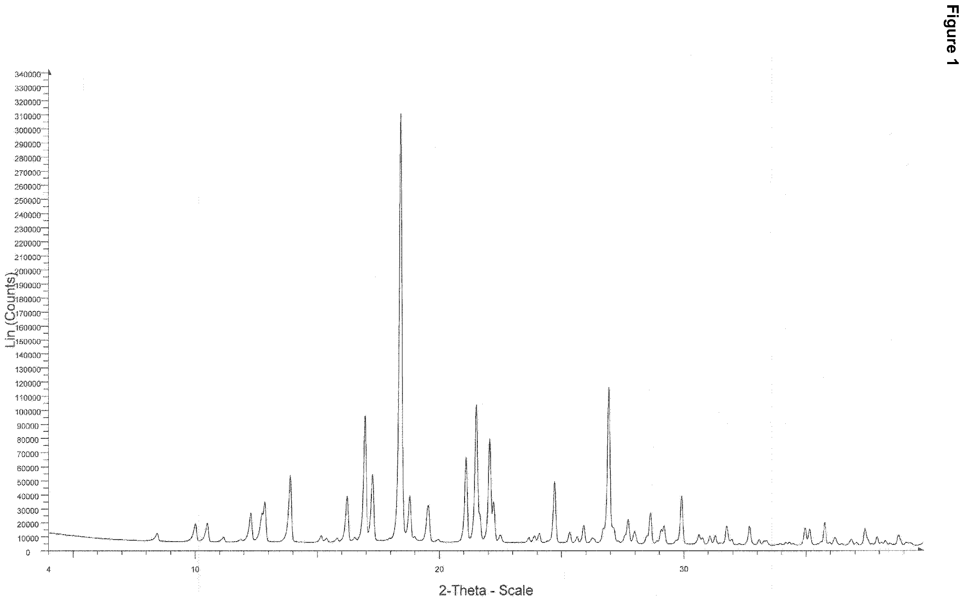

Figure 1 shows the XPRD analysis of apixaban, polymorph N-1.

### DETAILED DESCRIPTION OF THE INVENTION

The first aspect of the present invention refers to an efficient process for the preparation of apixaban, 1-(4-methoxyphenyl)-7-oxo-6-[4-(2-oxopiperidin-1-yl)phenyl]-4,5,6,7-tetrahydro-1*H*-pyrazolo[3,4-c]pyridine-3-carboxamide (compound I), or a pharmaceutically acceptable salt thereof, wherein the process comprises at least the following steps:
a) Contacting ethyl 6-(4-(5-bromopentanamido)phenyl)-1-(4-methoxyphenyl)-7-oxo-4,5,6,7-tetrahydro-1*H*-pyrazolo[3,4-*c*]pyridine-3-carboxylate (compound II) with formamide in the presence of a base to yield 1-(4-methoxyphenyl)-7-oxo-6-(4-(2-oxopiperidin-1-yl)phenyl)-4,5,6,7-tetrahydro-1*H*-pyrazolo[3,4-*c*]pyridine-3-carboxamide (compound I); 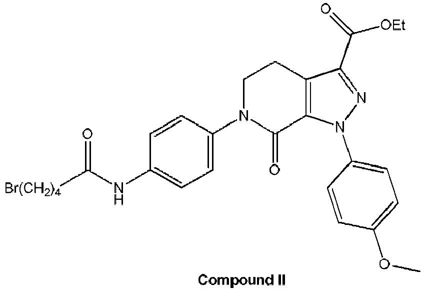 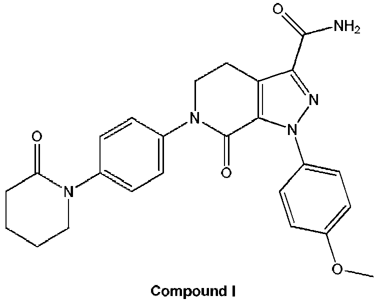 wherein the ratio of base to compound II, is from 2:1 (mol/mol) to 10:1 (mol/mol). The process provides apixaban in high purity and yield and can be easily performed at industrial scale.
   The 6-(4-(5-bromopentanamido)phenyl)-1-(4-methoxyphenyl)-7-oxo-4,5,6,7-tetrahydro-1*H*-pyrazolo[3,4-*c*]pyridine-3-carboxylate (compound II) is contacted with formamide in the presence of a base to yield 1-(4-methoxyphenyl)-7-oxo-6-(4-(2-oxopiperidin-1-yl)phenyl)-4,5,6,7-tetrahydro-1*H*-pyrazolo[3,4-*c*]pyridine-3-carboxamide (compound I). The mixture can also be stirred to ease the formation of the compound I.
   The ratio of the base to compound II must be comprised between 2:1 (mol/mol) and 10:1 (mol/mol), thus from 2 mols of base per mol of compound II to 10 mol of base per mol of compound II should be used. Preferably, the ratio is from 2:1 (mol/mol) to 5:1 (mol/mol). Most preferably, the ratio is from 2.5:1 to 3:1, most preferably 2.8:1, yielding apixaban in higher yields and purity.
   Suitable alkoxide bases include linear or branched alkoxides of metals (metal alkoxides), ammonium (ammonium alkoxides), boron (boron alkoxides) and silicon (silicon alkoxides). Metal alkoxides include, linear or branched alkoxides of metals selected from the group consisting of alkaline metals, alkaline earth metals transition metals and mixtures thereof. In an embodiment of the present invention the alkoxide base is a linear or branched C₁-C₁₂ alkoxide of metals selected from the group consisting of alkaline metals, alkaline earth metals and mixtures thereof. Preferably, a linear or branched C₁-C₅ alkoxide of an alkaline metal or alkaline earth metal may be used. More preferably, a linear or branched C₁-C₅ alkoxide of an alkaline metal or alkaline earth metal may be used. In another embodiment the alkoxide base is an ammonium alkoxide base. Non-limiting examples of alkoxide bases are sodium methoxide, sodium ethoxide, sodium propoxide, sodium isopropoxide, sodium butoxide, sodium isobutoxide, sodium sec-butoxide, sodium tert-butoxide, lithium methoxide, lithium ethoxide, potassium methoxide, potassium ethoxide, potassium propoxide, potassium isopropoxide, potassium butoxide, potassium isobutoxide, potassium sec-butoxide, and potassium tert-butoxide, calcium methoxide, calcium ethoxide, magnesium methoxide, magnesium ethoxide, barium methoxide, barium ethoxide, aluminum methoxide, aluminum ethoxide, titanium methoxide, titanium ethoxide, zirconium methoxide, zirconium ethoxide, ammonium methoxide, ammonium ethoxide, silicon methoxide, silicon ethoxide, boron methoxide, boron ethoxide. Among them sodium methoxide, sodium ethoxide, potassium methoxide and potassium ethoxide are preferred.
   The reaction is carried out over a range of temperatures from 0 °C to 50 °C. Preferably, the reaction temperature range is from 0 °C to 20 °C. Most preferably, between 0 °C and 10 °C, as the purity of apixaban is improved.
   The formamide of step a) may constitute the reaction media. Optionally, the reaction can also be carried out in the presence of an organic solvent as a reaction media. Suitable organic solvents may be used for the present invention include, but are not limited to: from hydrocarbon solvents, such as n-pentane, n-hexane, n-heptane, n-octane, paraffin, cyclohexane, methylcyclohexane, decahydronaphthalene, mineral oil, crude oils; aromatic hydrocarbon solvents, such as benzene, toluene, o-xylene, m-xylene, and p-xylene); halogenated hydrocarbon solvents, such as carbon tetrachloride, 1,2-dichloroethane, dichloromethane, chloroform; ester solvents, such as ethyl formate, methyl acetate, ethyl acetate, isopropyl acetate, ethyl malonate; ketone solvents, such as acetone, methyl ethyl ketone, cyclohexanone, cyclopentanone; ether solvents, such as diethyl ether, dipropyl ether, diphenyl ether, tetrahydrofuran, 1,4-dioxane; amine solvents, such as propyl amine, diethylamine, triethylamine, aniline, pyridine; alcohol solvents, such as methanol, ethanol, 1-propanol, 1-butanol, 1-octanol, benzyl alcohol, phenol, trifluoroethanol, glycerol, ethylene glycol, propylene glycol, m-cresol; nitrobenzene, *N*,*N*-dimethylformamide, *N*,*N-*dimethylacetamide, dimethyl sulfoxide, N-methyl-2-pyrrolidone, acetonitrile; silicone solvents, such as silicone oils, polysiloxanes, cyclosilicones. In an embodiment of the present invention the organic solvent is a polar organic solvent and preferably, the organic solvent is selected from the group consisting of N,N-dimethylformamide, C₁-C₄ alkyl alcohols, C₁-C₄-alkyl acetates and mixtures thereof. In a more preferred embodiment the organic solvent is selected from the group consisting of tetrahydrofuran, methyltetrahydrofuran, toluene, *N*,*N*-dimethylformamide, ethyl acetate and mixtures thereof. More preferably, the organic solvent is *N*,*N*-dimethylformamide. In a particular embodiment, the organic solvent may be formed by the combination of two or more organic solvents. The use of an organic solvent, as a reaction media, facilitates the reaction and also the precipitation of the apixaban.
   The intermediate compound II, 6-(4-(5-bromopentanamido)phenyl)-1-(4-methoxyphenyl)-7-oxo-4,5,6,7-tetrahydro-1*H*-pyrazolo[3,4-*c*]pyridine-3-carboxylate, may be obtained as described in WO2007/001385. Advantageously, the process of the present invention reduces significantly the percentage of impurity 1-(4-methoxyphenyl)-7-oxo-6-(4-(2-oxopiperidin-1-yl)phenyl)-4,5,6,7-tetrahydro-1*H*-pyrazolo[3,4-c]pyridine-3-carboxylic acid.
b) Isolating the apixaban obtained in step a),
c) optionally purifying the apixaban of step a) or b) by means of conventional purification techniques.
   Preferably the apixaban is isolated by precipitation and filtration, or also removing the solvent by distillation.
   Apixaban is obtained in very high purity and yields. Yields of the reaction are as good as 80 %, and the purity is always very high, being as good as 99.5 %.
   The disclosure also provides a process for the preparation of apixaban polymorph N-1, as described in patent WO2007/001385 and in figure 1, wherein the apixaban obtained in step a) or step b) is dissolved or suspended in an organic solvent with stirring at reflux temperature. The organic solvent is methanol. Afterwards, the resulting solution or dispersion is cooled to room temperature. The crystals obtained are isolated by means of conventional isolation techniques, preferably by filtration. Optionally, the obtained apixaban, polymorph N-1, may be further purified or dried or both. The term "room temperature" in the context of the preparation of apixaban polymorph N-1 means that the temperature is between 15-30 °C.
   Advantageously, the process is easy reproducible at an industrial scale with low energy and costs. In addition, the product is obtained in high yields and high polymorphic and HPLC purity.
d) Optionally, converting the apixaban obtained in step a) or b) into a pharmaceutically acceptable salt or co-crystal thereof.
   Salts or co-crystals of apixaban, can be prepared by contacting apixaban with an pharmaceutical acceptable acid at temperature between -10 °C and 100 °C.

Advantageously, the process of the present invention reduces the number of synthetic steps to obtain apixaban and is easily reproducible at an industrial scale with low energy, since it is carried out a relatively low temperature and costs. In addition, this process provides apixaban in high yields and high HPLC purity.

In a preferred embodiment the process for the manufacturing of apixaban, or a pharmaceutically acceptable salt thereof, the process comprises the following steps:
a) Contacting ethyl 6-(4-(5-bromopentanamido)phenyl)-1-(4-methoxyphenyl)-7-oxo-4,5,6,7-tetrahydro-1*H*-pyrazolo[3,4-*c*]pyridine-3-carboxylate (compound II) with formamide in the presence of a base, selected from alkaline metal of a linear or branched C₁-C₅ alkoxide to yield apixaban; 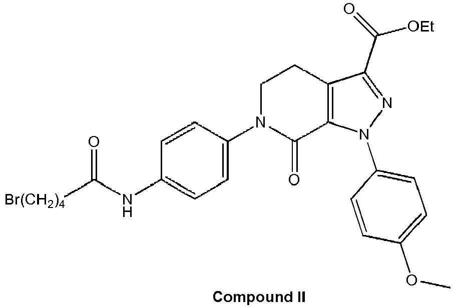 wherein the ratio of base to compound II, is from 2:1 (mol/mol) to 5:1 (mol/mol).
b) isolating the apixaban obtained in step a)
c) and optionally purifying apixaban of step a) or b) by means of conventional purification techniques.

In a further preferred embodiment the process for the manufacturing of apixaban, or a pharmaceutically acceptable salt thereof, the process comprises the following steps:
a) Contacting ethyl 6-(4-(5-bromopentanamido)phenyl)-1-(4-methoxyphenyl)-7-oxo-4,5,6,7-tetrahydro-1*H*-pyrazolo[3,4-*c*]pyridine-3-carboxylate (compound II) with formamide in the presence of a base, selected from alkaline or alkaline earth metals of linear or branched C₁-C₅ alkoxides and also in the presence of an organic solvent to yield apixaban; 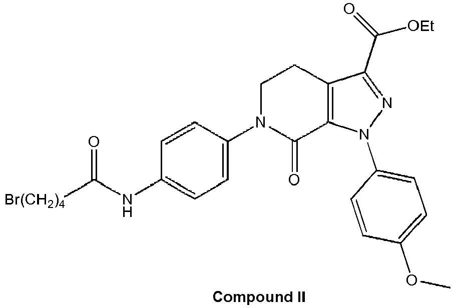 wherein the ratio of base to compound II, is from 2:1 (mol/mol) to 5:1 (mol/mol).
b) isolating the apixaban obtained in step a)
c) and optionally purifying apixaban of step a) or b) by means of conventional purification techniques.

In a further preferred embodiment the process for the manufacturing of apixaban, or a pharmaceutically acceptable salt thereof, the process comprises the following steps:
a) Contacting ethyl 6-(4-(5-bromopentanamido)phenyl)-1-(4-methoxyphenyl)-7-oxo-4,5,6,7-tetrahydro-1*H*-pyrazolo[3,4-*c*]pyridine-3-carboxylate (compound II) with formamide in the presence of an ammonium alkoxide base and also in the presence of an organic solvent to yield apixaban; 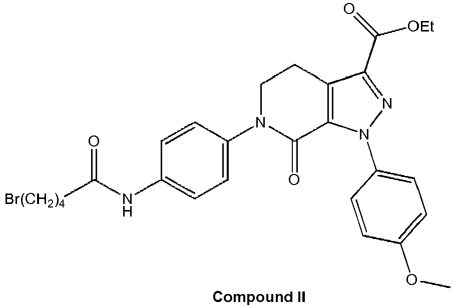 wherein the ratio of base to compound II, is from 2:1 (mol/mol) to 5:1 (mol/mol).
b) isolating the apixaban obtained in step a)
c) and optionally purifying apixaban of step a) or b) by means of conventional purification techniques.

In a further preferred embodiment the process for the manufacturing of apixaban, or a pharmaceutically acceptable salt thereof, the process comprises the following steps:
a) Contacting ethyl 6-(4-(5-bromopentanamido)phenyl)-1-(4-methoxyphenyl)-7-oxo-4,5,6,7-tetrahydro-1*H*-pyrazolo[3,4-*c*]pyridine-3-carboxylate (compound II) with formamide in the presence of a base, selected from alkaline or alkaline earth metals of linear or branched C₁-C₅ alkoxides, at a temperature between 0-50 °C to yield apixaban; 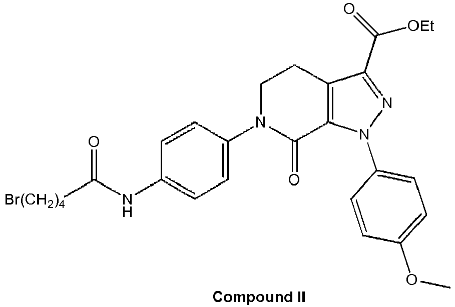 wherein the ratio of base to compound II, is from 2:1 (mol/mol) to 5:1 (mol/mol).
b) isolating the apixaban obtained in step a)
c) and optionally purifying apixaban of step a) or b) by means of conventional purification techniques.

In a further preferred embodiment the process for the manufacturing of apixaban, or a pharmaceutically acceptable salt thereof, the process comprises the following steps:
a) Contacting ethyl 6-(4-(5-bromopentanamido)phenyl)-1-(4-methoxyphenyl)-7-oxo-4,5,6,7-tetrahydro-1H-pyrazolo[3,4-c]pyridine-3-carboxylate (compound II) with formamide in the presence of a base, selected from alkaline or alkaline earth metals of linear or branched C₁-C₅ alkoxides, at a temperature between 0-10 °C to yield apixaban; 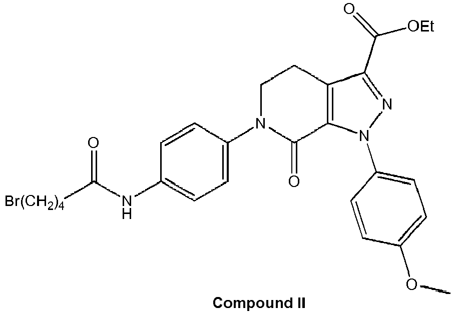 wherein the ratio of base to compound II, is from 2:1 (mol/mol) to 5:1 (mol/mol).
b) isolating the apixaban obtained in step a)
c) and optionally purifying apixaban of step a) or b) by means of conventional purification techniques.

A second aspect of the present disclosure relates to a pharmaceutical composition comprising a therapeutically effective amount of apixaban prepared according to the first aspect of the present invention together with appropriate amount of pharmaceutically acceptable excipients or carriers.

A third aspect of the present disclosure relates to the pharmaceutical composition of the second aspect of the present disclosure for use in a medicament.

A forth aspect of the present disclosure relates to the pharmaceutical composition of the second aspect of the present disclosure to prevent venous thromboembolism in adults following a hip or knee replacement operation and/or to prevent stroke and blood clots in other organs in adults with atrial fibrillation

In the following, the present invention is further illustrated by examples. Unless indicated otherwise, all indications of percentage are by weight and temperatures are in degrees Celsius.

### EXAMPLES

### Example 1: Preparation of 1-(4-methoxyphenyl)-7-oxo-6-[4-(2-oxopiperidin-1-yl)phenyl]-4,5,6,7-tetra hydro-1H-pyrazolo[3,4-c]pyridine-3-carboxamide (Apixaban).

To a mixture of ethyl 6-(4-(5-bromopentanamido)phenyl)-1-(4-methoxyphenyl)-7-oxo-4,5,6,7-tetrahydro-1*H*-pyrazolo[3,4-c]pyridine-3-carboxylate (50 g, 88 mmol) in 200 mL DMF and 89 mL (2.237 mmol) formamide was added an already prepared solution of sodium methoxide 30 % in methanol (47 mL, 247 mmol) at temperature between 0 - 5 °C. The resulting reaction mixture was stirred for 2 hours at a temperature between 0-5°C. Afterwards, 400 mL of water were added to the reaction mixture and the resulting suspension was stirred for 2 hours. The solid obtained was filtered and washed with 25 mL of water yielding 114.7 g of wet solid. The solid is dried yielding 37 g of apixaban. Polymorph H2-2 (as described in WO2007/001385) was obtained.
Yield: 85 %.
Purity (HPLC): 99.4 %.
DSC: Peaks with onsets at 55 °C (-101 J/g), 149 °C (-9 °C J/g), 168 °C (97 J/g) and 232 °C (-87 J/g).
¹H-NMR (200 MHz, DMSO) (2500) δ: 1.74-1.84 (m, 4H); 2.38-2.49 (m, 2H); 3.20-3.22 (m, 2H); 3.59-3.60 (m, 2H); 3.80 (s, 3H); 4.01-4.05 (m, 2H); 6.97-7.52 (m, 8H, NH); 7.74 (s, 1H, NH).

The solid obtained was suspended in 600 mL methanol and the dispersion was heated to the reflux temperature of the solvent for 1 hour. The dispersion was then cooled down to 23 - 25 °C and left under stirring for 1 hour at this temperature. The resulting suspension was then cooled down to 0 - 5 °C and left under stirring for 1 hour. The white solid was filtered and washed with 25 mL methanol. The product obtained was dried under vacuum at 40 °C to a constant weight, yielding 33 g of the titled compound. Polymorph N-1 (as described in WO2007/001385) was obtained.
Yield: 96 %
Purity (HPLC): 99.5 %.
DSC: Peak with an onset at 237 (-132 J/g).
PXRD (power x-ray diffraction) of Polymorph N-1 correspond to figure 1.
¹H-NMR (200 MHz, DMSO) (2500) δ: 1.74-1.84 (m, 4H); 2.38-2.49 (m, 2H); 3.20-3.22 (m, 2H); 3.59-3.60 (m, 2H); 3.80 (s, 3H); 4.01-4.05 (m, 2H); 6.97-7.52 (m, 8H, NH); 7.74 (s, 1H, NH).

### Example 2: Preparation of 1-(4-methoxyphenyl)-7-oxo-6-[4-(2-oxopiperidin-1-yl)phenyl]-4,5,6,7-tetrahydro-1H-pyrazolo[3,4-c]pyridine-3-carboxamide (Apixaban).

To a mixture of ethyl 6-(4-(5-bromopentanamido)phenyl)-1-(4-methoxyphenyl)-7-oxo-4,5,6,7-tetrahydro-1*H*-pyrazolo[3,4-c]pyridine-3-carboxylate (10 g, 17.5 mmol) in 50 ml (1.257 mmol) formamide an already prepared solution of sodium methoxide 30 % in methanol (11 mL, 58 mmol) was added at 0 - 5 °C. The resulting reaction mixture was stirred for 16 h at 23 - 25 °C. Afterwards, 100 mL water were added to the mixture and the resulting suspension was stirred for 2 hours at 0 - 5 °C. The solid was filtered and washed with 5 mL of water. The solid is dried yielding 7.5 g of apixaban. Polymorph H2-2 (as described in WO2007/001385) was obtained.
Yield: 86 %.
Purity (HPLC): 99.3 %.
DSC: Peaks with onsets at 55 °C (-101 J/g), 149 °C (-9 °C J/g), 168 °C (97 J/g) and 232 °C (-87 J/g).
¹H-NMR (200 MHz, DMSO) (2500) δ: 1.74-1.84 (m, 4H); 2.38-2.49 (m, 2H); 3.20-3.22 (m, 2H); 3.59-3.60 (m, 2H); 3.80 (s, 3H); 4.01-4.05 (m, 2H); 6.97-7.52 (m, 8H, NH); 7.74 (s, 1H, NH).

The crystalline solid obtained was suspended in 120 mL methanol, and the dispersion was heated to reflux temperature of the solvent for 1 hour. The dispersion was then cooled down to 23 - 25 °C and left under stirring for 1 hour at this temperature. The resulting suspension was then cooled to 0 - 5 °C and left under stirring for 1 hour. The white solid was filtered and washed with 5 mL of methanol. The obtained solid corresponds to crystalline form N-1 (as described in WO2007/001385 and figure 1) was dried at 40 °C to a constant weight (6.7 g).
Yield: 96 %
Purity (HPLC): exceeds over 99 %.
¹H-NMR (200 MHz, DMSO) (2500) δ: 1.74-1.84 (m, 4H); 2.38-2.49 (m, 2H); 3.20-3.22 (m, 2H); 3.59-3.60 (m, 2H); 3.80 (s, 3H); 4.01-4.05 (m, 2H); 6.97-7.52 (m, 8H, NH); 7.74 (s, 1H, NH).

## Claims

1. A process for the manufacturing of apixaban, or a pharmaceutically acceptable salt thereof, wherein the process comprises at least the following steps:
a) Contacting ethyl 6-(4-(5-bromopentanamido)phenyl)-1-(4-methoxyphenyl)-7-oxo-4,5,6,7-tetrahydro-1*H*-pyrazolo[3,4-*c*]pyridine-3-carboxylate (compound II) with formamide and a base to yield apixaban; 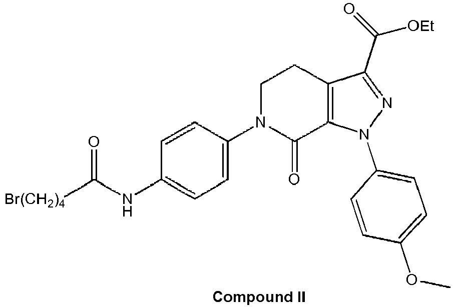 wherein the ratio of base to compound II, is from 2:1 (mol/mol) to 10:1 (mol/mol).
b) isolating the apixaban obtained in step a)
c) optionally, purifying apixaban of step a) or b) by means of conventional purification techniques.
d) optionally, converting the apixaban obtained in step a) or b) into a pharmaceutically acceptable salt or co-crystal thereof.

2. A process for preparing apixaban according to claim 1, wherein the reaction of step a) is carried out in the presence of an organic solvent.

3. A process according to claim 2, wherein the organic solvent of step a) is a polar organic solvent.

4. A process according to claim 3, wherein the organic solvent of step a) is selected from *N*,*N*-dimethylformamide, C₁-C₄ alkyl alcohols, C₁-C₄-alkyl acetates and their mixtures

5. A process according to claim 4, wherein the organic solvent used in step a) is *N*,*N-*dimethylformamide.

6. A process for preparing apixaban according to any of the preceding claims, wherein the base of step a) is selected from the group consisting of metal alkoxides, boron alkoxides, silicon alkoxides, ammonium alkoxides and mixtures thereof.

7. A process for preparing apixaban according to claim 6, wherein the base is a linear or branched alkoxide of metals selected from the groups consisting of alkaline metals, alkaline earth metals, transition metals and mixtures thereof.

8. A process for preparing apixaban according to claim 7, wherein the base of step a) is a linear or branched C₁-C₁₂ alkoxide of metals selected from the group consisting of alkaline metals, alkaline earth metals and mixtures thereof.

9. A process for preparing apixaban according to according to claim 8, wherein the base of step a) is a linear or branched C₁-C₅ alkoxide of an alkaline metal or alkaline earth metals.

10. A process for preparing apixaban according to according to claim 9, wherein the base of step a) is a linear or branched C₁-C₅ alkoxide of an alkaline metal.

11. A process for preparing apixaban according to according to claim 10, wherein the base of step a) is sodium methoxide, sodium ethoxide, potassium methoxide or potassium ethoxide.

12. A process for preparing apixaban according to any of the preceding claims, wherein the ratio of base to compound II, is from 2:1 (mol/mol) to 5:1 (mol/mol).

13. A process for preparing apixaban according to claim 12, wherein the ratio of base to compound II is from 2.5:1 to 3:1.

14. A process according to any of the preceding claims, wherein the reaction of step a) is carried out at a temperature between 0 °C and 50 °C.

15. A process according to claim 14, wherein the reaction of step a) is carried out at a temperature between 0 °C and 10 °C.

## Patentansprüche

1. Verfahren zum Herstellen von Apixaban oder eines pharmazeutisch annehmbaren Salzes desselben, wobei das Verfahren wenigstens die folgenden Schritte umfasst:
a) Kontaktieren von Ethyl-6-(4-(5-brompentanamido)phenyl)-1-(4-methoxyphenyl)-7-oxo-4,5,6,7-tetrahydro-1*H*-pyrazolo[3,4-*c*]pyridin-3-carboxylat (Verbindung II) mit Formamid und einer Base, um Apixaban zu ergeben: 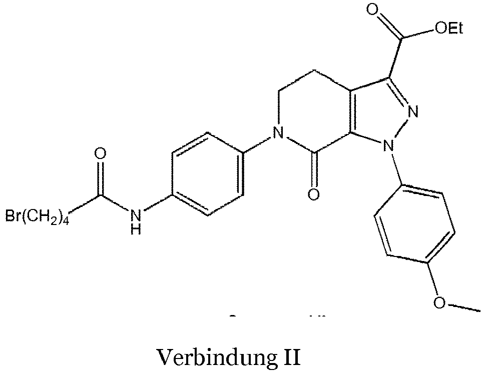 wobei das Verhältnis von Base zu Verbindung II von 2:1 (mol/mol) bis 10:1 (mol/mol) ist,
b) Isolieren des in Schritt a) erhaltenen Apixabans,
c) optional Reinigen von Apixaban aus Schritt a) oder b) mittels herkömmlicher Reinigungsmethoden,
d) optional Umwandeln des in Schritt a) oder b) erhaltenen Apixabans in ein pharmazeutisch annehmbares Salz oder einen Co-Kristall desselben.

2. Verfahren zum Herstellen von Apixaban nach Anspruch 1, wobei die Reaktion von Schritt a) in der Gegenwart eines organischen Lösungsmittels durchgeführt wird.

3. Verfahren nach Anspruch 2, wobei das organische Lösungsmittel aus Schritt a) ein polares organisches Lösungsmittel ist.

4. Verfahren nach Anspruch 3, wobei das organische Lösungsmittel aus Schritt a) ausgewählt wird aus N,N-Dimethylformamid, C₁-C₄-Alkylalkoholen, C₁-C₄-Alkylacetaten und deren Mischungen.

5. Verfahren nach Anspruch 4, wobei das in Schritt a) verwendete organische Lösungsmittel N,N-Dimethylformamid ist.

6. Verfahren zum Herstellen von Apixaban nach einem der vorangehenden Ansprüche, wobei die Base aus Schritt a) ausgewählt wird aus der Gruppe bestehend aus Metallalkoxiden, Boralkoxiden, Siliciumalkoxiden, Ammoniumalkoxiden und Mischungen derselben.

7. Verfahren zum Herstellen von Apixaban nach Anspruch 6, wobei die Base ein lineares oder verzweigtes Alkoxid von Metallen ausgewählt aus den Gruppen bestehend aus Alkalimetallen, Erdalkalimetallen, Übergangsmetallen und Mischungen derselben ist.

8. Verfahren zum Herstellen von Apixaban nach Anspruch 7, wobei die Base aus Schritt a) ein lineares oder verzweigtes C₁-C₁₂-Alkoxid von Metallen ausgewählt aus der Gruppe bestehend aus Alkalimetallen, Erdalkalimetallen und Mischungen derselben ist.

9. Verfahren zum Herstellen von Apixaban nach Anspruch 8, wobei die Base aus Schritt a) ein lineares oder verzweigtes C₁-C₅-Alkoxid eines Alkalimetalls oder Erdalkalimetalls ist.

10. Verfahren zum Herstellen von Apixaban nach Anspruch 9, wobei die Base aus Schritt a) ein lineares oder verzweigtes C₁-C₅-Alkoxid eines Alkalimetalls ist.

11. Verfahren zum Herstellen von Apixaban nach Anspruch 10, wobei die Base aus Schritt a) Natriummethoxid, Natriumethoxid, Kaliummethoxid oder Kaliumethoxid ist.

12. Verfahren zum Herstellen von Apixaban nach einem der vorangehenden Ansprüche, wobei das Verhältnis von Base zu Verbindung II von 2:1 (mol/mol) bis 5: (mol/mol) ist.

13. Verfahren zum Herstellen von Apixaban nach Anspruch 12, wobei das Verhältnis von Base zu Verbindung II von 2,5:1 bis 3:1 ist.

14. Verfahren nach einem der vorangehenden Ansprüche, wobei die Reaktion aus Schritt a) bei einer Temperatur zwischen 0°C und 50°C durchgeführt wird.

15. Verfahren nach Anspruch 14, wobei die Reaktion aus Schritt a) bei einer Temperatur zwischen 0°C und 10°C durchgeführt wird.

## Revendications

1. Un procédé de fabrication d'apixaban, ou un sel pharmaceutiquement acceptable de celui-ci, où le procédé comprend au moins les étapes suivantes :
a) mettre en contact le 6-(4-(5-bromopentanamido)phényl)-1-(4-méthoxyphényl)-7-oxo-4,5,6,7-tétrahydro-1*H*-pyrazolo[3,4-*c*]pyridine-3-carboxylate d'éthyle (composé II) avec du formamide et une base afin d'obtenir de l'apixaban ; 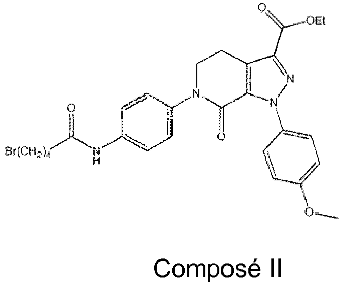 où le rapport de la base au composé II est de 2:1 (mol/mol) à 10:1 (mol/mol).
b) isoler l'apixaban obtenu à l'étape a)
c) éventuellement, purifier l'apixaban de l'étape a) ou b) au moyen de techniques de purification conventionnelles.
d) éventuellement, convertir l'apixaban obtenu à l'étape a) ou b) en un sel ou co-cristal pharmaceutiquement acceptable de celui-ci.

2. Un procédé de préparation d'apixaban selon la revendication 1, où la réaction de l'étape a) est effectuée en présence d'un solvant organique.

3. Un procédé selon la revendication 2, où le solvant organique de l'étape a) est un solvant organique polaire.

4. Un procédé selon la revendication 3, où le solvant organique de l'étape a) est sélectionné parmi *N*,*N*-diméthylformamide, alcools d'alkyle en C₁-C₄, acétates d'alkyle en C₁-C₄ et leurs mélanges.

5. Un procédé selon la revendication 4, où le solvant organique utilisé à l'étape a) est *N*,*N*-diméthylformamide.

6. Un procédé de préparation d'apixaban selon l'une quelconque des revendications précédentes, où la base de l'étape a) est sélectionnée parmi le groupe constitué d'alcoxydes de métal, d'alcoxydes de bore, d'alcoxydes de silicium, d'alcoxydes d'ammonium et de mélanges de ceux-ci.

7. Un procédé de préparation d'apixaban selon la revendication 6, où la base est un alcoxyde linéaire ou ramifié de métaux sélectionnés parmi les groupes constitués de métaux alcalins, de métaux alcalino-terreux, de métaux de transition et de mélanges de ceux-ci.

8. Un procédé de préparation d'apixaban selon la revendication 7, où la base de l'étape a) est un alcoxyde linéaire ou ramifié en C₁-C₁₂ de métaux sélectionnés parmi le groupe constitué de métaux alcalins, de métaux alcalino-terreux, et de mélanges de ceux-ci.

9. Un procédé de préparation d'apixaban selon la revendication 8, où la base de l'étape a) est un alcoxyde linéaire ou ramifié en C₁-C₅ d'un métal alcalin ou de métaux alcalino-terreux.

10. Un procédé de préparation d'apixaban selon la revendication 9, où la base de l'étape a) est un alcoxyde linéaire ou ramifié en C₁-C₅ d'un métal alcalin.

11. Un procédé de préparation d'apixaban selon la revendication 10, où la base de l'étape a) est du méthanolate de sodium, de l'éthanolate de sodium, du méthanolate de potassium ou de l'éthanolate de potassium.

12. Un procédé de préparation d'apixaban selon l'une quelconque des revendications précédentes, où le rapport de la base au composé II est de 2:1 (mol/mol) à 5:1 (mol/mol).

13. Un procédé de préparation d'apixaban selon la revendication 12, où le rapport de la base au composé II et de 2,5:1 à 3:1.

14. Un procédé selon l'une quelconque des revendications précédentes, où la réaction de l'étape a) est effectuée à une température entre 0 °C et 50 °C.

15. Un procédé selon la revendication 14, où la réaction de l'étape a) est effectuée à une température entre 0 °C et 10 °C.
